# EUROPÄISCHE PATENTSCHRIFT

(11) **EP 2 268 245 B1**
(45) Veröffentlichungstag und Bekanntmachung des Hinweises auf die Patenterteilung: **17.07.2013**
(21) Anmeldenummer: 09734098.8
(22) Anmeldetag: 21.04.2009
(51) Int. Cl.: A61F 13/06, A61F 13/10

(54) **ORTHOPÄDISCHES HILFSMITTEL MIT EINBRINGBAREM FUNKTIONSELEMENT**
ORTHOPAEDIC AID WITH INSERTABLE FUNCTIONAL ELEMENT
PRODUIT D'AIDE ORTHOPÉDIQUE AVEC UN ÉLÉMENT FONCTIONNEL À INTRUDUIRE

(30) Priorität: 21.04.2008 DE 202008005533 U
(43) Veröffentlichungstag der Anmeldung: 05.01.2011
(73) Patentinhaber: medi GmbH & Co. KG, 95448 Bayreuth (DE)
(72) Erfinder: WEIHERMÜLLER, Stefan, 95448 Bayreuth (DE)
(74) Vertreter: Blaumeier, Jörg
(86) Internationale Anmeldenummer: PCT/DE2009/000511
(87) Internationale Veröffentlichungsnummer: WO 2009/129780

(56) Entgegenhaltungen:
- EP-A1- 0 496 071
- US-A- 4 870 956
- US-A- 5 421 811
- US-A- 6 029 277
- US-A1- 2005 197 608

## Beschreibung

Die vorliegende Erfindung betrifft ein orthopädisches Hilfsmittel, eine Bandage, Orthese, Schiene, Stütze, ein kompressives Beckleidungs- oder Gestrickteil oder dergleichen zur Therapie oder Unterstützung von entsprechenden Körperteilen, insbesondere solche Hilfsmittel die als Strickteil ausgeführt sind.

Orthopädische Hilfsmittel haben sich seit Jahren bewährt und werden neben der therapeutischen Behandlung von Beschwerden, wie auch postoperativ auch als Stütz-, Schutz- oder Unterstützungsmittel für entsprechende Körperteile eingesetzt. US 2005/197608 offenbart eine Handgelenkbandage zur Fixierung eines Handgelenks. US 4870956 zeigt eine Kniebandage, und US 6029277 einen therapeutischen Handschuh.

Insbesondere Bandagen für verschiedene Körperteile, wie z.B. Kniegelenke, Ellenbogen- oder Handgelenke werden bei Gelenkentzündungen und/oder nach einer Verletzung oder präventiv bei verschiedenen Aktivitäten, wie z.B. Sport getragen um die entsprechenden Gelenke zu schonen, zu unterstützen oder diese vor erneuter Verletzung oder zu hoher Beanspruchung zu schützen.

Es hat sich gezeigt, dass für verschiedene Anwendungsfälle und für unterschiedliche Körperteile sehr unterschiedliche und wechselnde Anforderungen an solche Hilfsmittel gestellt werden.

Ist etwa bei einer Person z.B. die Stützung des Knies an den Außenseite des Beinverlaufes wünschenswert, so kann dies in anderen Fällen oder zu einem anderen Zeitpunkt, zur Rückgewinnung der Mobilität bei der gleichen Person unvorteilhaft sein, wie z.B. bei vorangeschrittener Genesung, wenn durch eine zu starke Stützung durch die Bandage der Muskel- Wiederaufbau verzögert wird.

Grundsätzlich sind verschiedene Bandagen verfügbar die auch auf solche wechselnden Bedürfnisse zugeschnitten sind, wie z.B. Kniebandagen mit oder ohne eingearbeitete Pelotten für die Kniescheibe oder mit eingenähten seitlichen Versteifungen um z.B. die Beugung des Kniegelenk einzuschränken oder seitliche Belastung zu vermindern. Nach dem gleichen Prinzip sind auch Bandagen für das Handgelenk bekannt, welche über entsprechende Versteifungen verfügen, durch welche z.B. ein Verdrehen oder Überbeugen des Handgelenkes vermieden wird.

Die genannten Versteifungen sind dabei an den entsprechenden Stellen in die Bandagen oder andere Hilfsmittel eingearbeitet und mit der Bandage verbunden. Oft werden daher von einer Person verschieden Bandagen benötigt um verschiedene Grade der Unterstützungswirkung abzudecken, wie z.B. eine stärker stützende Kniebandage nach einer Operation, welche im Anschluss zum besseren Aufbau der Muskulatur mit einer weniger Stützenden Wirkung benötigt wird.

Wünschenswert wären daher Bandagen, bzw. Hilfsmittel, welche über einstellbare oder änderbare Stützwirkung und zusätzliche Wirkung verfügen, wie z.B. eine zusätzliche therapeutische oder medizinische Funktion.

Aufgabe der vorliegenden Erfindung ist es, ein orthopädisches Hilfsmittel zu schaffen, welches den wechselnden Bedürfnissen in Bezug auf variabler Unterstützungswirkung und zusätzlicher therapeutischer oder medizinischer Funktion gerecht wird.

Diese Aufgabe wird mit den Merkmalen des kennzeichnenden Teils des Anspruchs 1 gelöst. Fortbildungen und vorteilhafte Ausführungen der Erfindung sind im Folgenden umfasst.

Erfindungsgemäß ist ein Orthopädisches Hilfsmittel wie eine Bandage, Orthese, Schiene, Stütze, Bekleidungs- oder Gestrickteil oder dergleichen zur Therapie oder Unterstützung von entsprechenden Körperteilen mit einem Funktionselement mit zusätzlicher therapeutischer Wirkung, wobei das orthopädische Hilfsmittel ein kompressives Strickteil ist, welches wenigstens eine Aufnahmevorrichtung für das zusätzliche Funktionselement und wenigstens eine in das Strickteil eingearbeitete Öffnung als Zugang zu der Aufnahmevorrichtung aufweist, wobei die Öffnung die Kompressionseigenschaften des Hilfsmittel nicht nachteilig verändert, und wobei die Öffnung als im Strickprozess eingearbeiteter Schlitz in Querrichtung des Gestricks ausgeführt ist.

Das Aufnahmeteil ist bevorzugt eine Tasche für das zusätzliche Funktionselement, welche beispielsweise auf der Außenseite der z.B. Bandage oder Schiene angeordnet ist, welche über der Öffnung in dem orthopädischen Hilfsmittel angeordnet ist und dort etwa aufgenäht, aufgeschweißt oder aufgeklebt ist. Der Zugang zu dieser aufgebrachten Aufnahmevorrichtung oder Tasche wird durch die Öffnung in dem Strickteil beispielhaft von Innen her ermöglicht, wobei das zusätzliche Funktionselement durch diese Öffnung in die Tasche eingebracht wird und leicht wieder entfern-oder wechselbar ist. Dadurch können z.B. verschiedene unterstützende Funktionselemente im Wechsel leicht eingebracht werden und für eine Behandlung unterschiedliche Anwendungen realisieren, bei welchen das betroffene Körperteil mehr oder weniger stark durch ein entsprechendes Funktionselement unterstütz wird.

Diese Mittel können je nach Anforderung verschiedene Härten, bzw. Steifigkeiten aufweisen um dadurch unterschiedlich starke Stütz oder Schutzfunktionen zu übernehmen. Es wird damit möglich, z.B. ein Kniegelenk direkt nach einer Operation durch Tragen einer solchen Bandage mit einem sehr steifen Zusatz relativ ruhig zu stellen und diese Ruhigstellung nach einer gewissen Genesung, durch Ersetzen des Einsatzes aus einem weniger steifen Material, zu vermindern um die Rückgewinnung der Mobilität zu unterstützen. In dem Gelenk kann dadurch der Muskelaufbau unterstütz werden, welcher bei konstantem Tragen einer Bandage mit einem sehr steifen Einsatz deutlich verzögert erfolgen würde.

Die erfindungsgemäßen Bandagen oder dergleichen verfügen bevorzugt über eine, in das Strickteil eingearbeitete, nicht ausfransende Öffnung bzw. Loch und eine auf einer Seite der Bandage aufgebrachte Tasche bzw. Aufnahmevorrichtung für das zusätzliche Mittel, welche in Form und Größe den zu verwendeten Mitteln angepasst ist. Die Öffnung ist dabei in Querrichtung des Strickteils als Schlitz eingebracht, welcher im Strickprozess ausgebildet wird, wodurch der Öffnungsrand ohne zusätzliche Verstärkung oder erforderliches Abnähen des Randes direkt nicht ausfransend ausgebildet ist. Durch diese stricktechnische Maßnahme bei der Ausbildung der Öffnung erhält man zum einen eine optimale Öffnung für das zusätzliche Funktionselement und das Strickteil behält dadurch seine kompressiven Eigenschaften, z.B. bei einer Bandage für das Handgelenk bezüglich ihres Umfanges, was bei einer Öffnung in Längsrichtung nicht der Fall wäre, da die Öffnung den Kompressionsverlauf unterbrechen würde. Auch ein Ausfransen einer z.B. nur durch Ausschneiden oder Ausstanzen realisierten Öffnung würde im Verlauf der Benutzung die kompressiven Eigenschaften eines solchen, als Strickteil ausgeführten orthopädischen Hilfsmittels nachteilig beeinflussen.

Eine Aufnahmetasche ist dabei auf einer Seite der Bandage aufgenäht, aufgeklebt oder anders dauerhaft befestigt und kann durch die in der Bandage eingearbeitete Öffnung erreicht werden.

Durch die Öffnung kann nun das zusätzliche Funktionsteil in die Tasche eingeschoben werden. Die Öffnung und die Aufnahmetasche sind derart zueinander angeordnet, dass die Tasche beidseitig über die Öffnung hinausragt und ein Funktionsteil zunächst durch die Öffnung in Richtung der längeren Seite der Tasche eingeschoben wird und das restliche Teil des Funktionsteils durch leichtes Dehnen der Bandage vollständig in die Tasche eingeschoben werden kann. Das Funktionsteil erstreckt sich nach seinem Einsatz also über die Öffnung und kann damit während des Tragens der Bandage nicht versehentlich herausgleiten.

Das Funktionsteil, wie z.B. ein Versteifungsstab aus Kunststoff, Metall, Glas-, Kohlefaser oder anderen Materialien kann über die Öffnung, bevorzugt auf der Innenseite der Bandage leicht herausgenommen werden und durch einen anderen ersetzt werden oder komplett entfernt werden.

Neben dem Einsatz von Funktionsteilen zur Unterstützung der verschiedenen Körperteile können auch Funktionselemente, etwa mit thermischer oder anderen Wirkungen wie z.B. medizinischer Wirkung zum Einsatz kommen. Diese können z.B. als Stäbe oder Gelpacks mit kühlender oder wärmender Wirkung zum Einsatz kommen, womit Entzündungen oder Schwellungen in den Gelenken behandelt werden können. Auch entsprechende Einsätze mit Wirkstoffen, welche in Gelenknähe an diese abgegebenen werden sind und eine medizinische oder therapeutische Wirkung haben können so unkompliziert zum Einsatz kommen. Nach einer weiteren Variante können auch Funktionsteile mit zusätzlichen stimulierenden Elementen, wie z.B. Massagenoppen oder elektrisch stimulierenden Elementen eingesetzt werden. Auch diese können jederzeit entfernt und/oder gewechselt werden.

Im Folgenden wird die Erfindung anhand von Zeichnungen beispielhaft näher beschrieben. Dabei zeigen:
- Fig. 1: Vorderansicht einer Handgelenkbandage mit Loch
- Fig. 2: Vorderansicht einer Handgelenkbandage mit Öffnungsschlitz
- Fig. 3: Vorderansicht einer Handgelenkbandage, getragen mit Aufnahmetasche
- Fig. 4: perspektivische Ansicht einer Kniebandage

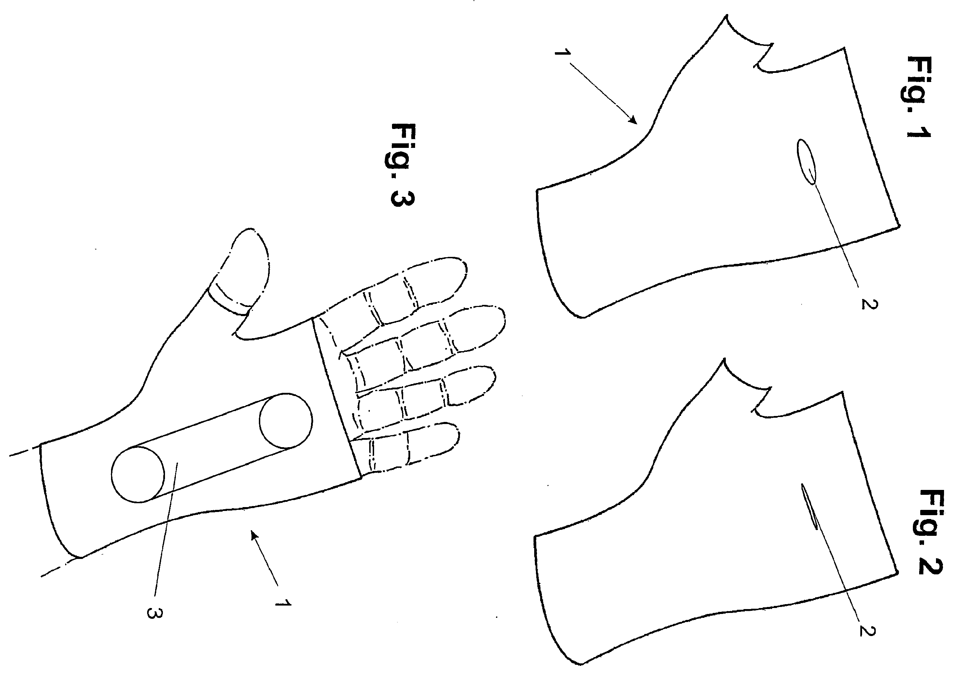

In Fig. 1 ist eine erfindungsgemäße Bandage 1 für ein Handgelenk gezeigt, welche die beschriebene Öffnung 2 aufweist, welche hier, zur Veranschaulichung, leicht aufgeweitet dargestellt ist. Die nicht aufgeweitete Öffnung in der Bandage ist in der Fig. 2 gezeigt. Über diese Durchführung 2 wird eine Tasche 3, wie in Fig. 3 gezeigt, aufgesetzt, welche bevorzugt auf der Außenseite des orthopädischen Hilfsmittels aufgebracht ist.

Die Tasche ist optisch mit oben und unten begrenzenden Kreisen dargestellt, was eine reine Gestaltungsvariante darstellt und keine Öffnungen darstellt. In diese Tasche 3 kann nun, wie hier beispielhaft dargestellt, von der Innenseite der Handgelenkbandage her, ein zusätzliches therapeutisches oder medizinisches Mittel durch die Öffnung 2 in die Tasche 3 eingeschoben werden.

Bevorzugt werden hier steife Stäbchen eingesetzt um z.B. die Beugebewegung der Hand zu verhindern um diese für eine erforderliche Zeit ruhig zu stellen.

Nach Abheilung der Verletzung kann das, in die Tasche 3 eingeschobene steife Stäbchen, dann entfernt werden oder gegen ein entsprechend weicheres ausgetauscht werden, um den Grad der Ruhigstellung zu verändern.

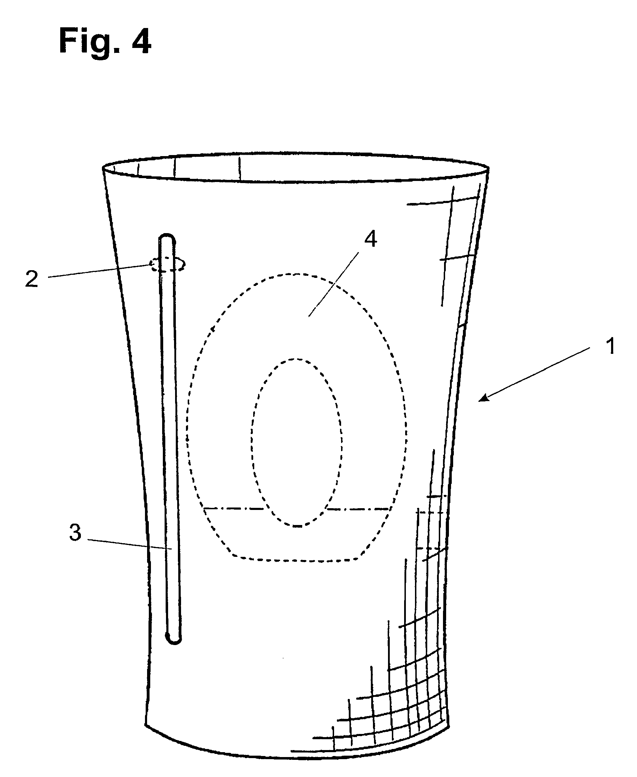

Fig. 4 zeigt eine Kniebandage 1, welche über eine bereits eingearbeitete Pelotte 4 für die Kniescheibe verfügt und welche auch mit einer an der Außenseite der Bandage aufgebrachten Aufnahmetasche 3 ausgeführt ist. Auch hier kann ein Stab durch die Öffnung 2 von der Innenseite der Bandage her, in die Aufnahmetasche 3 eingeschoben werden. Da die Tasche 3 und der einzuschiebende Stab in Richtung oben, über die Öffnung 2 herausragen, wird der Stab durch leichte Dehnung der Bandage 1 komplett in die Tasche eingeschoben und ragt daher in der Tasche über die Öffnung 2 hinweg, wodurch er in der Tasche fixiert ist.

Nachdem bevorzugte Ausführungen der Erfindung in Bezug auf die beiliegenden Zeichnungen beschrieben wurden, ist festzuhalten, dass die Erfindung nicht auf diese genauen Ausführungen und Anwendungsfälle beschränkt ist und dass verschiedene Änderungen und Modifizierungen daran von einem Fachmann ausgeführt werden können, ohne dass vom Umfang der Erfindung, wie er in den beiliegenden Ansprüchen definiert ist abgewichen wird.

## Patentansprüche

1. Orthopädisches Hilfsmittel wie Bandage, Orthese, Schiene, Stütze, kompressives Bekleidungs-, bzw. Gestrickteil oder dergleichen zur Therapie oder Unterstützung von entsprechenden Körperteilen mit einem Funktionselement mit zusätzlicher therapeutischer Wirkung,
**dadurch gekennzeichnet,**
**dass** das orthopädische Hilfsmittel (1) ein kompressives Strickteil ist, welches wenigstens eine Aufnahmevorrichtung (3) für das zusätzliche Funktionselement und wenigstens eine in das Strickteil eingearbeitete Öffnung als Zugang zu der Aufnahmevorrichtung (3) aufweist, wobei die Öffnung die Kompressionseigenschaften des Hilfsmittels nicht negativ verändert,
wobei die Öffnung (2) als im Strickprozess eingearbeiteter Schlitz in Querrichtung des Gestricks ausgeführt ist.

2. Orthopädisches Hilfsmittel nach Anspruch 1
**dadurch gekennzeichnet,**
**dass** das orthopädische Hilfsmittel (1) mit wenigstens einer Tasche (3) ausgerüstet ist, in welche Funktionselemente mit zusätzlicher therapeutischer Wirkung wechselbar einbringbar sind.

3. Orthopädisches Hilfsmittel nach Anspruch 1
**dadurch gekennzeichnet,**
**dass** der Eingriff, bzw. die Öffnung als nicht ausreißende oder ausfransende Öffnung (2) in dem Gestrick ausgeführt ist und auf einer Seite des Gestricks (1) eine Tasche (3) für das zusätzliche Funktionselement angebracht ist.

4. Orthopädisches Hilfsmittel nach Anspruch 4,
**dadurch gekennzeichnet,**
**dass** die Tasche (3) für das zusätzliche Funktionselement aufgenäht, aufgeschweißt oder aufgeklebt ist.

5. Orthopädisches Hilfsmittel nach Anspruch 1
**dadurch gekennzeichnet,**
**dass** das Funktionselement mit zusätzlicher therapeutischer Wirkung eine Temperatur beeinflussende Wirkung aufweist.

6. Orthopädisches Hilfsmittel nach Anspruch 1
**dadurch gekennzeichnet,**
**dass** das Funktionselement mit zusätzlicher therapeutischer Wirkung eine medizinische Wirkung aufweist.

7. Orthopädisches Hilfsmittel nach Anspruch 1
**dadurch gekennzeichnet,**
**dass** das Funktionselement mit zusätzlicher therapeutischer Wirkung als wirkender Festkörper ausgeführt ist.

8. Orthopädisches Hilfsmittel nach Anspruch 1
**dadurch gekennzeichnet,**
**dass** das Funktionselement mit zusätzlicher therapeutischer Wirkung als wirkende Gel- oder wenigstens teilelastische Pelotte ausgeführt ist.

9. Orthopädisches Hilfsmittel nach Anspruch 1
**dadurch gekennzeichnet,**
**dass** das Funktionselement mit zusätzlicher therapeutischer Wirkung als wirkender elastischer Formkörper ausgeführt ist.

## Claims

1. An orthopedic aid such as a bandage, orthotic device, splint, support, compressive garment, knitted part or similar for therapy or support of corresponding body parts wtih a functional element with additional therapeutic action,
**characterised in that**
the orthopedic aid (1) is a compressive knitted part which provides at least one receiving device (3) for the additional functional element and at least one opening worked into the knitted part as an access to the receiving device (3), wherein the opening does not negatively change the compressive properties of the aid, whereas the opening (2) is embodied as a slit worked into the transverse direction of the knit in the knitting process.

2. The orthopedic aid according to claim 1,
**characterised in that**
the orthopedic aid (1) is fitted with at least one pocket (3) into which functional elements with additional therapeutic action can be introduced in a replaceable manner.

3. The orthopedic aid according to claim 1,
**characterised in that**
the access or the opening is embodied in the knit as a non-tearing or non-fraying opening (2), and a pocket (3) for the additional functional element is attached to one side of the knit.

4. The orthopedic aid according to claim 4,
**characterised in that**
the pocket (3) for the additional functional element is sewn on, welded on or glued on.

5. Thge orthopedic aid according to claim 1,
**characterised in that**
the functional element with additional therapeutic action provides a temperature-influencing action.

6. The orthopedic aid according to claim 1,
**characterised in that**
the functional element with additional therapeutic action provides a medicinal action.

7. The orthopedic aid according to claim 1,
**characterised in that**
the functional element with additonal therapeutic action is embodied as a solid body.

8. The orthopedic aid according to claim 1,
**characterised in that**
the functional element with additional therpeutic action is embodied as an active gel pad or an at least partially elastic pad.

9. The orthopedic aid according to claim 1,
**characterised, in that**
the functional element with additional therapeutic action is embodied as an active, elastic formed body.

## Revendications

1. Dispositif orthopédique comme un bandage, une orthèse, une glissière, une attelle, un vêtement ou un tricot compressif ou autre pour le traitement ou l'appui de parties correspondantes du corps avec un élément fonctionnel avec une action thérapeutique supplémentaire,
**caractérisé en ce que**
le dispositif orthopédique (1) est un tricot compressif qui comprend au moins un dispositif de logement (3) pour l'élément fonctionnel supplémentaire et au moins une ouverture aménagée dans la pièce de tricot pour accéder au dispositif de logement (3), l'ouverture ne modifiant pas de manière négative les propriétés de compression du dispositif orthopédique, l'ouverture (2) étant conçue comme une fente réalisée, lors du processus de tricotage, dans la direction transversale du tricot.

2. Dispositif orthopédique selon la revendication 1
**caractérisé en ce que**
le dispositif orthopédique (1) est muni d'au moins une poche (3) dans laquelle des éléments fonctionnels avec une action thérapeutique supplémentaire peuvent être introduits de manière amovible.

3. Dispositif orthopédique selon la revendication 1
**caractérisé en ce que**
l'intervention ou l'ouverture est réalisée comme une ouverture (2) non déchirable ou sans formation de franges dans le tricot et, sur un côté du tricot (1) est prévue une poche(3) pour l'élément fonctionnel supplémentaire.

4. Dispositif orthopédique selon la revendication 4
**caractérisé en ce que**
la poche (3) pour l'élément fonctionnel supplémentaire est cousu, soudé ou collé.

5. Dispositif orthopédique selon la revendication 1
**caractérisé en ce que**
l'élément fonctionnel avec une action thérapeutique supplémentaire a une action influençant la température.

6. Dispositif orthopédique selon la revendication 1
**caractérisé en ce que**
l'élément fonctionnel avec une action thérapeutique supplémentaire a une action médicale.

7. Dispositif orthopédique selon la revendication 1
**caractérisé en ce que**
l'élément fonctionnel avec une action thérapeutique supplémentaire est réalisé comme un corps solide actif.

8. Dispositif orthopédique selon la revendication 1
**caractérisé en ce que**
l'élément fonctionnel avec une action thérapeutique supplémentaire est réalisé comme une pelote active de gel ou au moins partiellement élastique.

9. Dispositif orthopédique selon la revendication 1
**caractérisé en ce que**
l'élément fonctionnel avec une action thérapeutique supplémentaire est réalisé comme un corps moulé actif élastique.
